Europäisches Patentamt

European Patent Office

Office européen des brevets

(11) Veröffentlichungsnummer: **0 173 837**
**A2**

# EUROPÄISCHE PATENTANMELDUNG

(21) Anmeldenummer: 85108989.6

(22) Anmeldetag: 18.07.85

(51) Int. Cl.⁴: **A 61 D 17/00**

(30) Priorität: 01.09.84 DE 3432281

(43) Veröffentlichungstag der Anmeldung:
12.03.86 Patentblatt 86/11

(84) Benannte Vertragsstaaten:
AT BE CH DE FR GB IT LI NL SE

(71) Anmelder: Richard Herberholz KG
Höfen 82a
D-5600 Wuppertal 2(DE)

(72) Erfinder: Neutsch, Karl-Heinz
Leiken
D-5650 Solingen 1(DE)

(74) Vertreter: Buse, Karl Georg, Dipl.-Phys. et al,
Patentanwälte Dipl.-Phys. Buse Dipl.-Phys. Mentzel
Dipl.-Ing. Ludewig Unterdörnen 114
D-5600 Wuppertal 2(DE)

(54) Schallkopf für Echolot-Geräte zur Trächtigkeitsuntersuchung bei Tieren.

(57) Bei einem Schallkopf für Echolot-Geräte zur Trächtigkeit-suntersuchung bei Tieren, der über ein Kabel mit dem Testgerät (Ultraschallgerät) verbunden ist, ist ein flaches mit einem abgerundeten Vorderteil ausgerüstetes Gehäuse vorgesehen, das den Sende- und Empfangs-Schallwandler in einer seitlichen Aufnahme im Gehäuse trägt, wobei der Schallkopf von einem Ultraschallstrahlen durchlässigen, das Kontaktmittel in der Arbeitslage sichernden Umhüllung umgeben ist. Als Umhüllung des Schallkopfes kann eine Überzugskappe oder ein ärmellanger Einmalhandschuh aus Gummi oder Kunststoff dienen. Das Unterteil des Schall-kopfes kann ein Kupplungsglied für eine Verlängerungsvor-richtung tragen. Diese kann aus einem oder mehreren Kunststoffrohren bestehen.

EP 0 173 837 A2

./...

FIG.1

PATENTANWÄLTE: 0173837

DIPL.-PHYS. BUSE · DIPL.-PHYS. MENTZEL · DIPL.-ING. LUDEWIG

Unterdörnen 114 · Postfach 200210 · 5600 Wuppertal 2 · Fernruf (0202) 557022/23/24 · Telex 8591606 wpat

68                                       **5600 Wuppertal 2, den**

                                         Kennwort: "Flach-Schallkopf"


Richard Herberholz KG.,
5600 Wuppertal 2, Höfen 82 a

---

Schallkopf für Echolot-Geräte zur Trächtigkeitsuntersuchung bei Tieren

---

Die Erfindung bezieht sich auf einen Schallkopf für Echolot-Geräte zur Trächtigkeitsuntersuchung bei Tieren. Die bekannten Echolot-Geräte haben sich zur Trächtigkeitsdiagnose bei Schweinen weitgehend durchgesetzt. Von den Schallköpfen der Testgeräte werden Ultraschallwellen ausgesetzt, die beim Auftreffen auf Trennflächen zwischen verschiedenen Geweben oder zwischen Geweben und Flüssigkeit zurückgeworfen und von den Schallköpfen, die gleichzeitig als Empfänger dienen, an die Testgeräte weitergeleitet werden, wo die Erzeugung von akustischen oder visuellen Signalen stattfindet. Bei Schweinen erfolgen die Messungen an der Bauchdecke und zwar eine Hand breit vor dem Hinterbein. Um einen einwandfreien Kontakt zwischen Schallkopf und Hautoberfläche zu erzielen, muß der Schallkopf mit Öl oder einem Gel beschichtet sein. Das vom Schallkopf ausgehende Ultraschallbündel kann nur einen begrenzten Bereich in der Bauchhöhle absuchen. Wenn dieser Bereich weit vorverlegt wird, kann zwar die sich in die Bauchhöhle absenkende Gebärmutter früher erfaßt werden, es besteht dann aber auch die Gefahr, daß andere Strukturen z.B. die gefüllte Harnblase durch Erzeugen eines Echos falsche Testergebnisse verursachen. Dieses ist der Grund, daß die bisher gebräuchlichen Echolot-Geräte zur Trächtigkeitsuntersuchung bei Rindern, Schafen und Ziegen ungeeignet sind. Bei Rindern ist die Fruchtblase, die zwischen dem 60 und 90 Tag nach der Befruchtung in der Bauchhöhle verschwindet, von den vom Schallkopf ausgesendeten Ultraschallbündeln kaum erreichbar. Die Messungen werden noch dadurch erschwert, daß die Haut der Rinder stark behaart ist, so daß ein guter Kontakt zwischen Schallkopf des Testgerätes und der Hautoberfläche der Rinder trotz Verwendung einer reichlichen Menge von Kontaktmitteln in Form von Öl oder Gel nicht zustande kommt. In eine günstigere Meßposition gelangt die Fruchblase bei Rindern erst nach dem 100 Tag der Befruchtung. Dieser Zeitpunkt ist aus wirtschaftlichen Gesichspunkten zu spät.

Bei der Trächtigkeitsuntersuchung von Schafen und Ziegen treten die gleichen Schwierigkeiten auf.

Es ist auch bekannt, eine Trächtigkeitsuntersuchung bei Rindern mit Ultraschallsonden durchzuführen, die in den Mastdarm eingeführt werden. Hierbei verursachen die blutführenden Gefäße im Bereich der Gebärmutter besondere Geräusche, die nach dem Doppler-Effekt bald hellere, bald dunklere Schattierungen aufweisen. Die Auswertung der Ton-Schattierungen ermöglichen einen Hinweis auf eine Trächtigkeit. Dieses bekannte Verfahren ist nicht nur sehr zeitaufwendig und wegen der erforderlichen Geräte auch kostspielig, sondern benötigt eine außerordentlich sorgfältige Durchführung, wenn brauchbare Meß-Ergebnisse erzielt werden sollen. Für unerfahrene und ungeübte Meßpersonen ist dieses Verfahren ungeeignet. Es wird deshalb in der Praxis abgelehnt.

Der Erfindung liegt die Aufgabe zugrunde, die bisher bekannten Testgeräte so zu verbessern, daß mit ihnen auch bei Rindern, Schafen und Ziegen zuverlässige Trächtigkeitsuntersuchungen durchgeführt werden können, ohne daß ihre Handlichkeit in irgendeiner Weise beeinträchtigt wird. Zur Lösung dieser Aufgabe wird erfindungsgemäß vorgeschlagen, daß der Schallkopf, welcher über ein Kabel mit dem Testgerät verbunden ist, ein flaches Gehäuse mit einem abgerundeten Vorderteil aufweist und den Sende- und Empfangs-Schallwandler in einer seitlichen Aufnahme im Gehäuse trägt, wobei der Schallkopf von einer Ultraschallstrahlen durchlässigen, das Kontaktmittel in seiner Arbeitslage sichernden Umhüllung umgeben ist. Hierdurch wird erreicht, daß der Schallkopf mit der Hand in den Mastdarm eines Rindes eingeführt und in eine Reichweite der Fruchtblase gebracht werden kann, daß zuverlässige Trächtigkeitsuntersuchungen möglich sind. Das Rückwandecho der mit Fruchtwasser gefüllten Fruchtblase ist deutlich festzustellen und zwar insbesondere dann, wenn der Sende- und Empfangs-Schallwandler in die Kontaktnähe der Fruchtblase gelangt. Das Einführen des Schallkopfes von Hand in den Mastdarm eines Rindes stellt heute für die Praktiker kein besonderes Problem dar.

Bei einer bevorzugten Ausführungsform der Erfindung dient als Umhüllung des Schallkopfes eine Überzugskappe oder ein ärmellanger Einmalhandschuh aus Gummi oder Kunststoff. Dieses ist aus hygienischen Gründen zweckmäßig.

Nach einem weiteren Merkmal der Erfindung kann das Gehäuse des Schallkopfes in seinem oberen Bereich zur versenkten Aufnahme des Sende- und Empfangs-Schallwandlers einen zylinderischen Hohlraum aufweisen, während das Unterteil des Schallkopfes ein Kupplungsglied für eine Verlängerungsvorrichtung trägt. Durch diese Ausbildung wird erreicht, daß der Schallkopf mit Hilfe der Verlängerungsvorrichtung in den Mastdarm eines Schafes oder einer Ziege eingeführt werden kann, weil in diesen Fällen das manuelle Einführen des Schallkopfes nicht möglich ist.

Die Verlängerungsvorrichtung kann aus einem oder mehreren Rohren, insbesondere aus Kunststoff bestehen. Bei der Verwendung von mehreren Rohren können diese vorteilhaft teleskopartig ineinanderschiebbar oder auseinanderziehbar ausgebildet sein. Zwischen der Verlängerungsvorrichtung und dem Kupplungsglied des Schallkopfes kann eine Schraub-, Klemm- oder eine andere lösbare Verbindung vorhanden sein. Auf der Verlängerungsvorrichtung können Markierungen angebracht sein, die Hinweise auf die richtige Gebrauchslage des Schallkopfes während der Trächtigkeitsuntersuchung von Rindern, Schafen und Ziegen enthalten.

Auf der Zeichnung ist die Erfindung in einem Ausführungsbeispiel dargestellt. Es zeigen:

Fig. 1    ein Rind in schaubildlicher Ansicht, teilweise im Schnitt, mit dem erfindungsgemäßen Gerät, dessen Schallkopf mit der Hand in den Mastdarm des Rindes eingeführt ist,

Fig. 2    ein Schaf in gleicher Darstellung wie in der Fig. 1, wobei sich der Schallkopf auf einer Verlängerungsvorrichtung befindet,

Fig. 3    das Verbindungskabel mit den elektrischen Anschlüssen und einem Teil der Verlängerungsvorrichtung,

Fig. 4    eine Vorderansicht des Schallkopfes, und

Fig. 5    eine Seitenansicht des Schallkopfes.

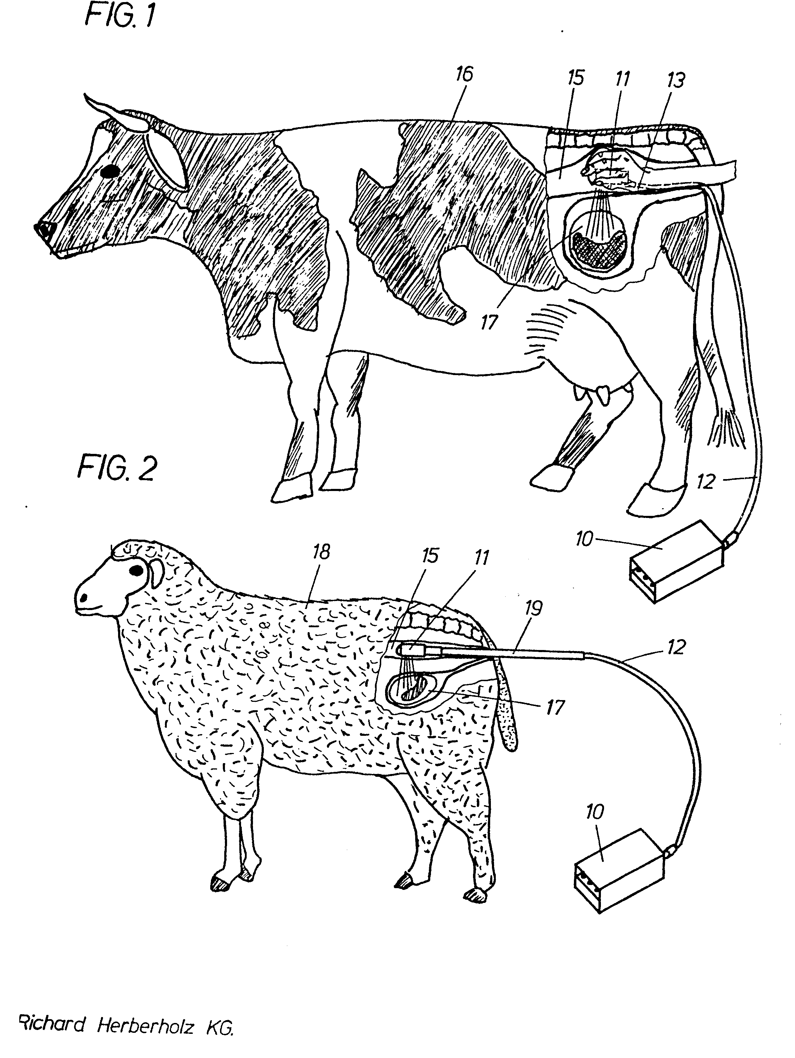

Das in den Fig. 1 und 2 dargestellte Testgerät ist ein handelsübliches Echolot-Gerät. Der erfindungsgemäße Schallkopf 11 steht über ein Kabel 12 mit dem Ultraschallgerät 10 in leitender Verbindung. Das Ultraschallgerät 10 wird mit wiederaufladbaren Akkus gespeist. Bei der Fig. 1 liegt der Schallkopf 11 in der Innenfläche einer Hand, die von einem ärmelartigen Einmalhandschuh 13 umgeben ist. Vor dem Einlegen wird der Schallkopf 11 mit Kontaktmittel 14 in Form von Öl oder Gel benetzt. Danach wird die Hand mit dem Schallkopf 11 in den Mastdarm 15 des Rindes 16 eingeführt und zwar so weit, bis die Finger die Fruchtblase 17 ertasten können. Die vom Schallkopf 11 ausgesandten Ultraschallbündel werden von der Fruchtblase 17 reflektiert und gelangen zu dem Schallkopf 11, der auch als Empfänger dient, zurück. Der Schallkopf 11 spricht nun das Testgerät 10 an, das bei einer festgestellten Trächtigkeit des Rindes 16 einen Dauerton erzeugt. Die Nichtträchtigkeit eines Rindes 16 wird vom Testgerät 10 durch einen unterbrochenen Ton angezeigt.

Bei Schafen 18 ist eine manuelle Einführung des Schallkopfes 11 in den Mastdarm 15 nicht möglich. In diesem Fall wird, genau wie bei Ziegen, der Schallkopf 11 auf eine Verlängerungsvorrichtung 19 gesetzt und in den Mastdarm 15 eingeführt. Die Trächtigkeitsuntersuchung geschieht in gleicher Weise wie bei dem Rind 16. Anstelle des Handschuhes wird eine Überzugskappe 13 aus Gummi oder Kunststoff verwendet. Dieses ist einmal aus hygienischen Gründen erforderlich und zum anderen deshalb, um ein Abwischen des Kontaktmittels 14 bei der Einführung zu verhindern.

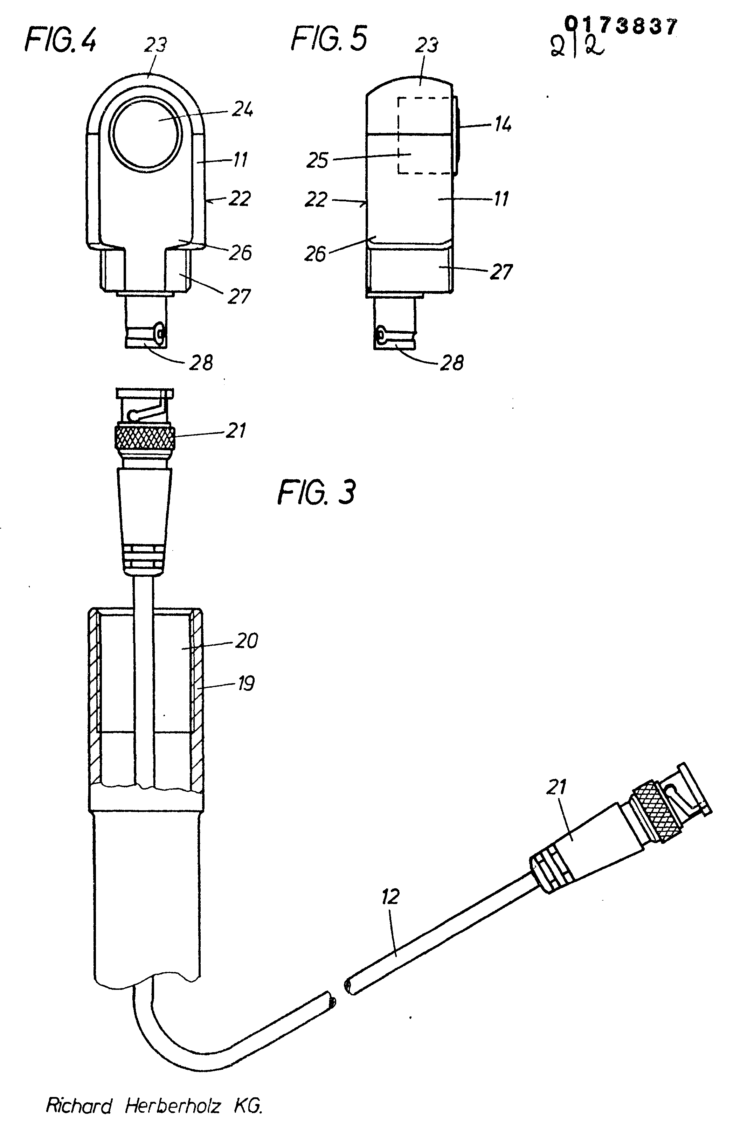

Wie insbesondere die Figur 3 veranschaulicht, besteht die Verlängerungsvorrichtung 19 aus einem Kunststoffrohr, in dessen Hohlraum 20 das Verbindungskabel 12 geführt ist. Die elektrischen Anschlüsse des Kabels 12 tragen das Bezugszeichen 21.

Der Schallkopf 11 ist in den Figuren 4 und 5 vergrößert dargestellt. Sein aus Kunststoff gefertigtes Gehäuse 22 ist flach gehalten und mit einem abgerundeten Vorderteil 23 bestückt. Der Sende- und Empfangs-Schallwandler 24 ist seitlich im Gehäuse 22 angeordnet und zwar versenkt in einem zylinderischen Hohlraum 25. Das Unterteil 26 des Schallkopfes 11 trägt ein Kupplungsglied 27, das mit der Verlängerungsvorrichtung 19 verbindbar ist. Bei dem gezeigten Ausführungsbeispiel besteht zwischen dem Kupplungsglied 27 und der Verlängerungsvorrichtung 19 eine Klemmverbindung. An dem Unterteil 26 sitzt ferner eine elektrische Steckdose 28, die den elektrischen Anschluß 21 des Kabels 12 aufnimmt.

Wie bereits erwähnt, ist die dargestellte Ausführungsform nur eine beispielsweise Verwirklichung der Erfindung. Diese ist nicht darauf beschränkt. Es sind noch mancherlei Abänderungen und Ausbildungen möglich. So könnte die Verlängerungsvorrichtung 19 aus teleskopartig ineinanderschiebbaren Rohren bestehen, die eine Längeneinstellung ermöglichen. Des weiteren könnte der Schallkopf 11 mit der Verlängerungsvorrichtung 19 verschraubt sein. Die Verlängerungsvorrichtung 19 könnte auch bei der Trächtigkeitsuntersuchung von Rindern 16 zur Anwendung kommen. Dieses könnte bei Jungtieren empfehlenswert sein.

**PATENTANWÄLTE**

**DIPL.-PHYS. BUSE · DIPL.-PHYS. MENTZEL · DIPL.-ING. LUDEWIG**

Unterdörnen 114 · Postfach 200210 · 5600 Wuppertal 2 · Fernruf (0202) 55 70 22/23/24 · Telex 8 591 606 wpat

68

5600 Wuppertal 2, den

Kennwort: "Flach-Schallkopf"

– 7 –

Bezugszeichenliste

| | |
|---|---|
| 10 | Echolotgerät, Ultraschallgerät |
| 11 | Schallkopf |
| 12 | Kabel |
| 13 | Umhüllung (Handschuh, Überzugskappe) |
| 14 | Kontaktmittel |
| 15 | Mastdarm |
| 16 | Rind |
| 17 | Fruchtblase |
| 18 | Schaf |
| 19 | Verlängerungsvorrichtung |
| 20 | Hohlraum der Verlängerungsvorrichtung 19 |
| 21 | elektrische Anschlüsse |
| 22 | Gehäuse des Schallkopfes 11 |
| 23 | Vorderteil des Schallkopfes 11 |
| 24 | Sende- und Empfangs-Schallwandler |
| 25 | zylinderischer Hohlraum im Gehäuse 22 |
| 26 | Unterteil des Schallkopfes 11 |
| 27 | Kupplungsglied des Schallkopfes 11 |
| 28 | Steckdose |

PATENTANWÄLTE  0173837

DIPL.-PHYS. BUSE · DIPL.-PHYS. MENTZEL · DIPL.-ING. LUDEWIG
Unterdörnen 114 · Postfach 200210 · 5600 Wuppertal 2 · Fernruf (0202) 55 70 22/23/24 · Telex 8 591 606 wpat

68                                      **5600 Wuppertal 2, den**
                                  Kennwort: "Flach-Schallkopf"


Richard Herberholz KG.,
5600 Wuppertal 2, Höfen 82 a

_____


                    A n s p r ü c h e :

1.) Schallkopf für Echolot-Geräte zur Trächtigkeitsunter-
    suchung bei Tieren, **d a d u r c h   g e k e n n -
    z e i c h n e t**, daß der Schallkopf (11), welcher
    über ein Kabel (12) mit dem Testgerät (10) verbunden ist, ein flaches Gehäuse (22) mit einem abgerundeten Vorderteil (23) aufweist und den Sende- und
    Empfangsschallwandler (24) in einer seitlichen Aufnahme (25) im Gehäuse (22) trägt, wobei der Schallkopf (11) von einer Ultraschallstrahlen durchlässigen,
    das Kontaktmittel (14) in seiner Arbeitslage sichernden Umhüllung (13) umgeben ist.


2.) Schallkopf nach Anspruch 1, dadurch gekennzeichnet,
    daß als Umhüllung des Schallkopfes (11) eine Überzugskappe oder ein ärmellanger Einmalhandschuh (13)
    aus Gummi oder Kunststoff dient.


3.) Schallkopf nach Anspruch 1 und 2, dadurch gekennzeichnet, daß das Gehäuse (22) des Schallkopfes (11) zur
    versenkten Aufnahme des Sende- und Empfangs-Schallumwandlers (24) einen zylinderischen Hohlraum (25)
    aufweist, während das Unterteil (26) des Schallkopfes
    (11) ein Kupplungsglied (27) für eine Verlängerungsvorrichtung (19) trägt.

4.) Schallkopf nach Anspruch 1 - 3, dadurch gekennzeichnet, daß die Verlängerungsvorrichtung (19) aus einem oder mehreren Rohren, insbesondere aus Kunststoff besteht.

5.) Schallkopf nach Anspruch 4, dadurch gekennzeichnet, daß die Rohre der Verlängerungsvorrichtung (19) teleskopartig ineinanderschiebbar oder auseinanderziehbar ausgebildet sind.

6.) Schallkopf nach Anspruch 1 - 5, dadurch gekennzeichnet, daß zwischen Verlängerungsvorrichtung (19) und dem Kupplungsglied (27) des Schallkopfes (11) eine Schraub-, Klemm- oder eine andere lösbare Verbindung vorgesehen ist.

7.) Schallkopf nach Anspruch 3 - 6, dadurch gekennzeichnet, daß an der Verriegelungsvorrichtung (19) Markierungen angebracht sind, die Hinweise auf die richtige Gebrauchslage des Schallkopfes (11) während der Trächtigkeitsuntersuchung von Rindern, Schafen und Ziegen enthalten.

FIG. 1

FIG. 2

Richard Herberholz KG.

FIG.4

FIG.5

0173837

2|2

FIG. 3

Richard Herberholz KG.